Europäisches Patentamt

European Patent Office

Office européen des brevets

(11) Veröffentlichungsnummer: **0 154 680 B1**

(12) **EUROPÄISCHE PATENTSCHRIFT**

(45) Veröffentlichungstag der Patentschrift:
18.01.89

(21) Anmeldenummer: **84113386.1**

(22) Anmeldetag: **07.11.84**

(51) Int. Cl.⁴: **A 61 F 5/01, A 61 F 13/06**

(54) Gelenkmanschette.

(30) Priorität: 29.09.84 DE 3435955
23.02.84 DE 8405572 U

(43) Veröffentlichungstag der Anmeldung:
18.09.85 Patentblatt 85/38

(45) Bekanntmachung des Hinweises auf die Patenterteilung:
18.01.89 Patentblatt 89/3

(84) Benannte Vertragsstaaten:
AT BE CH DE FR GB IT LI NL SE

(56) Entgegenhaltungen:
AT-B- 209 486
DE-U- 1 740 508
DE-U- 8 405 572
US-A- 1 465 233
US-A- 2 450 862
US-A- 3 584 622
US-A- 3 674 023
US-A- 4 280 489

(73) Patentinhaber: Grisar, Gunter, Dr., Am Südhang 3B,
D-6301 Wettenberg 3 (DE)

(72) Erfinder: Grisar, Gunter, Dr., Am Südhang 3B,
D-6301 Wettenberg 3 (DE)

(74) Vertreter: Missling, Arne, Dipl.-Ing. et al, Patentanwälte
Dipl.-Ing. R. Schlee Dipl.-Ing. A. Missling
Bismarckstrasse 43, D-6300 Giessen (DE)

ACTORUM AG

## Beschreibung

Die Erfindung betrifft eine Gelenkmanschette für die Stützung und Gelenkführung des Sprunggelenks bei sportlicher Betätigung, welche zur seitlichen Abdeckung des Sprunggelenks zwei Laschen aufweist, die über einen Mittelsteg derart miteinander verbunden sind, dass die Gelenkmanschette im ausgebreiteten Zustand eine leicht V-förmige Gestalt aufweist und dass bei angelegter Maschette das Schienbein sowie der Fussrücken frei bleiben, welche Manschette weiterhin einstückig und aus einem thermoplastischen Kunststoff gebildet ist und deren Laschen zur Befestigung am Bein oberhalb des Sprunggelenks mit einem Befestigungselement verbindbar sind.

Eine derartige Sprunggelenkmanschette ist z.B. aus der US-A-4 280 489 bekannt.

Bei Personen, welche eine Sprunggelenksdistorsion erlitten haben oder bei denen eine lockere Bandführung im oberen oder unteren Sprunggelenk vorliegt, oder solche, die wegen eines Bänderrisses operiert sind, oder aber auch für Personen, deren Sprunggelenke besonders verletzungsgefährdet sind, wie z.B. Fussballspieler, Handballspieler, Basketballspieler oder Volleyballspieler, ist die Stützung und Führung des Gelenks durch eine Gelenkmanschette erforderlich.

Eine Gelenkmanschette nach der US-A-4 280 489 ist jedoch nicht geeignet, um bei sportlichen Betätigungen eingesetzt zu werden, sei es nach Verletzungen oder aber, um Verletzungen von vorn herein auszuschalten. Diese bekannte Gelenkmanschette besteht aus zwei Schalen, die untereinander verbunden sind. Diese beiden Schalen sind starr ausgebildet, damit sie den Druck aufnehmen können, der von einem auf deren Innenseite angeordneten Luftbalg auf diese ausgeübt wird. Allein der Druck im Luftbalg ist für die Stabilität der Gelenkmanschette verantwortlich. Die beiden seitlichen Schalen sind über ein starres Teil miteinander verbunden, das bis unter die Ferse reicht. Hierdurch wird zum einen die Beweglichkeit der Manschette eingeengt und zum anderen ist die durch die Luftkammern gegebene Stabilität auch nicht ausreichend, um diese Manschette bei sportlicher Betätigung einzusetzen.

Aus der AT-B-2 109 486 ist ein Starrverband bekannt, der dazu dient, das Gelenk absolut stillzulegen. Eine sportliche Betätigung mit einem derartigen Starrverband ist ausgeschlossen. Aus dieser Druckschrift ist es bekannt, für den Starrverband ein Thermoplastisches Material zu verwenden, das bei Körpertemperatur starr ist und bei einer Temperatur, die nicht über 80°C, vorzugsweise bei 55 bis 60°C liegt erweicht und dadurch formbar wird. Aus dem DE-U-1 740 508 ist ein weiterer Starrverband bekannt, der aus einer Stütze bzw. einer Schiene aus einem Formstück eines thermoplastischen Kunststoffes besteht, der bei Körpertemperatur starr ist und bei einer Temperatur von nicht über 80°C erweicht und formbar wird. Sportliche Betätigungen lassen diese bekannten Starrverbände gleichfalls nicht zu.

Bekannte Gelenkmanschetten, die eine Bewegung des Gelenks zulassen, bestehen aus einem Gummistrumpf, der zum einen anatomisch geformt und kompressionsintensiv ist und so eine Passform gewährleistet. Der Nachteil eines derartigen Strumpfes besteht jedoch darin, dass er keinen Schutz gegen Distorsionen bietet und auch vor Gewalteinwirkungen auch kleinsten Ausmassen nicht schützt.

Bekannt ist es des weiteren, einen derartigen Gummistrumpf mit seitlichen über dem Sprunggelenk liegenden Spiralfedern zu verstärken, um eine Stabilitätserhöhung gegenüber dem Gummistrumpf zu erzielen. Allerdings ist der Stabilitätsgewinn unbedeutend, während die anderen Nachteile beibehalten werden.

Darüberhinaus sind Knöchelschnürbandagen bekannt, die aus Kunstleder mit seitlich gepolsterten Schienen und Fussrücken-Knöchelschnürung versehen sind. Im Bereich der Sprunggelenke sind elastische Schienen angeordnet, die zur Verstärkung dienen. Allerdings sind diese elastischen Mittel nicht dem jeweiligen individuellen Fussgelenk angepasst und bringen somit auch bei festem Schnüren keinen ausreichenden Stabilitätsgewinn.

Des weiteren sind Sportschuhe bekannt, in denen seitlich über des Sprunggelenk hochgezogene Verstärkungsleisten angebracht sind, die ein Umknicken beim Sport verhindern sollen. Diese Verstärkungsleisten bestehen aus einem elastischen Material, damit diese sich der individuellen Knöchelform anpassen können. Der Stabilitätsgewinn ist jedoch auch nur relativ gering, da die Anpassung der Verstärkungsleisten an die Fussform nur unvollkommen ist. Des weiteren liegt hier der Nachteil vor, dass ein spezieller Schuh erforderlich wird.

Des weiteren sind Tape-Verbände bekannt, die jedoch den Nachteil aufweisen, dass sie nur einmal verwandt werden und auch nicht über Schürfwunden angelegt werden können.

Der Erfindung liegt die Aufgabe zugrunde, eine Gelenkmanschette der eingangs genannten Art vorzuschlagen, die ein Umknicken des Sprunggelenks auch bei sportlicher Höchleistung mit Sicherheit verhindert, das Gelenk somit einwandfrei abstützt und in einfacher Weise dauerhaft dem individuellen Gelenk der zu behandelnden Person anpassbar ist und dabei die Bewegungsfähigkeit im oberen und unteren Sprunggelenk nicht einschränkt und auch Rotationsbewegungen zulässt und gleichzeitig als Knöchelschutz dient.

Diese Aufgabe wird mit den Merkmalen des kennzeichnenden Teiles des Anspruches 1 gelöst.

Eine erfindungsgemäss ausgebildete Gelenkmanschette für das Sprunggelenk besteht somit aus zwei Laschen, die seitlich über das Sprunggelenk gelegt werden. Diese beiden Laschen sind über einen Mittelsteg miteinander verbunden, der unter dem Fuss durchgezogen ist. Im ausgebreiteten Zustand der Gelenkmanschette ist diese leicht V-förmig ausgebildet, wobei die Breite des Mittelsteges etwas geringer ist als die Breite der Laschen. Die Breite der Laschen ist so gewählt, dass diese die Bewegungsfähigkeit im oberen und unteren Sprunggelenk nicht einschränkt. Die Gelenkmanschette besteht aus einem Niedertemperatur-Thermoplast, wobei vorteilhaft ein solches Thermoplast gewählt wird, das bereits bei möglichst geringen Temperaturen verformbar ist. Bekannt sind Thermoplaste, die sich bereits ab 55° verformen lassen. Für die Anpassung einer erfindungsgemässen Gelenkmanschette wird diese z.B. in ein Wasserbad von 60-70° gelegt und auf diese Temperatur angewärmt. Anschliessend wir die Manschette an den Fuss und das Sprunggelenk angedrückt und erhält somit die Negativform des Sprunggelenks und des Fusses, wobei das Niedertemperatur-Thermoplast eine einwandfrei negative Abbildung des individuellen Sprunggelenks oder auch jeden anderen Gelenks erhält. Jede Sprunggelenkmanschette ist somit der individuellen Fussform der Trägerperson angepasst. Für die Anlegung der Sprunggelenkmanschette werden die beiden Laschen am oberen Ende entweder über eine Binde am Unterschenkel festgelegt oder aber die Laschen werden untereinander über z.B. ein Klettband verbunden. Entscheidend für die hohe Steifigkeit und den sicheren Halt der Sprunggelenkmanschette ist somit, dass die beiden Laschen biegesteif über den Mittelsteg miteinander verbunden sind und ein formschlüssiges Anliegen an der Kontur des Fusses und des Gelenks gegeben ist, so dass auch bei grosser Krafteinwirkung auf das Sprunggelenk, wie sie beim Leistungssport eintreten kann, eine seitliche Beweglichkeit des Fusses relativ zur Manschette und damit ein Umknicken mit Sicherheit verhindert ist.

Für ein bequeneres Tragen der Sprunggelenkmanschette ist diese an der Innenseite mit einer Polsterschicht, z.B. einer Schaumstoffschicht, versehen. Die Manschette wird vorteilhaft auch mit einem Leder, Kunstleder oder einem Stoff überzogen, um dieser zum einen ein gefälligeres Aussehen zu geben und zum anderen die Polsterschicht abzudecken.

Eine Ausführungsform der Erfindung ist im folgenden anhand der Zeichnung näher beschrieben, in dieser zeigen:

Fig. 1 in perspektivischer Ansicht eine an einem Fuss befestigte Gelenkmanschette,

Fig. 2 eine Draufsicht auf die ausgebreitete Gelenkmanschette und

Fig. 3 einen Schnitt nach Linie III-III in Fig. 2.

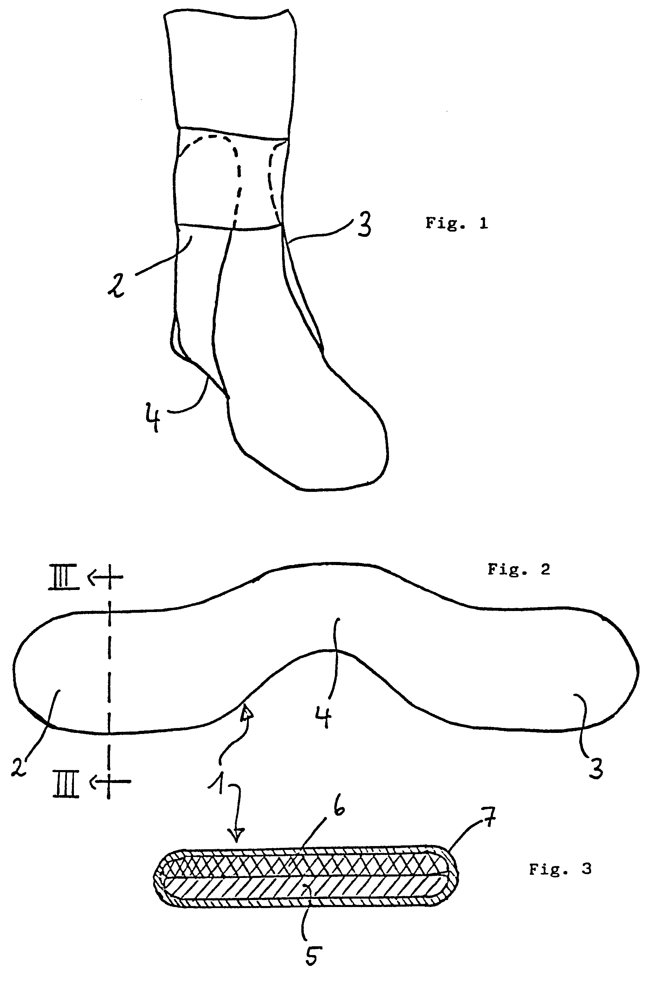

Die in den Figuren dargestellte Gelenkmanschette ist in Fig. 2 in ausgebreitetem Zustand gezeigt und weist eine im wesentlichen V-förmige Form auf, wobei die beiden Laschen 2, 3 der Sprunggelenkmanschette 1 mit einer etwas grösseren Breite als der Mittelsteg 4 ausgebildet sind, der die beiden Laschen miteinander verbindet. Die Sprunggelenkmanschette besteht aus einem einstückigen Streifen 5 aus einem Niedertemperatur-Thermoplast, der z.B. ab 55°C plastisch verformbar ist und nach der Abkühlung die erhaltene Form beibehält. Dieser Kunststoffstreifen 5 ist auf einer Seite mit einer Polsterschicht 6 versehen, die sich an das Gelenk anlegt und so das Tragen der Manschette 1 erleichtert. Der Streifen 5 sowie die Polsterschicht 6, die vorteilhaft aus einem Schaumstoff besteht, ist von einer Schutzhülle 7 umgeben, die z.B. aus Leder, Kunstleder oder aber aus Stoff bestehen kann. Diese Schicht dient zur Verbesserung des Aussehens der Gelenkmanschette und gleichzeitig zum Schutz der Polsterschicht 6.

In Fig. 1 ist die Gelenkmanschette 1 in angelegtem Zustand dargestellt. Die beiden Laschen 2, 3 sind seitlich über das Gelenk nach oben gezogen und übergreifen dieses. Diese beiden Laschen sind über den Mittelsteg 4 miteinander verbunden. Da die Gelenkmanschette aus einem thermoplastischen Kunststoff besteht, konnte diese des Fussform im Bereich des Mittelsteges 4 wie auch der Gelenke im Bereich der Laschen 2, 3 exakt angeformt werden. Zur Befestigung der Gelenkmanschette 1 am Fuss dient entweder, wie dies in Fig. 1 dargestellt ist, eine Binde, die zwei- oder dreimal um den Fuss und um die Laschen 2, 3 herumgewickelt ist oder aber ist es auch denkbar, einen Klettverschluss an der Aussenseite der Laschen 2, 3 anzubringen und die Verbindung durch ein Klettband vorzunehmen. Die Höhe der Gelenkmanschette ist so gewählt, dass diese im hinteren Drittel des Längsgewölbes in Höhe der Knöchelgabel das obere und untere Sprunggelenk umfasst. Die sehr gute Steifigkeit und Führung des Gelenks wird zum einen durch den Mittelsteg und zum anderen durch die formgerechte Anpassung der Gelenkmanschette erhalten.

Vorteilhaft wird ein Niedertemperatur-Thermoplast verwendet, das bei 55° bereits erweicht und plastisch verformbar ist. Die Laschen reichen an der Medialseite vorteilhaft bis ca. 10 cm oberhalb des Innenknöchels und an der Aussenseite bis ca. 11,5 cm.

Falls die Gelenkmanschette mit Kunststoff gepolstert und mit einet Hülle überzogen ist, so kann die Anpassung der Gelenkmanschette an das Gelenk auch mit der Kunststoffschicht und der Hülle erfolgen.

Eine erfindungsgemäss ausgebildete Gelenkmanschette kann darüber hinaus auch als integraler bestandteil in einen Schuh eingesetzt werden, wobei diese dann mit ihrem Mittelsteg an der Sohle des Schuhs befestigt wird. Die Anpassung erfolgt dann entweder durch Erwärmung des gesamten Schuhs oder aber mittels gezielter Erwärmung z.B. mittels eines Heissluftföns lediglich der Gelenkmanschette.

**Patentansprüche**

1. Gelenkmanschette für die Stützung und Gelenkführung des Sprunggelenks bei sportlicher Betätigung, welche zur seitlichen Abdeckung des Sprunggelenks zwei Laschen (2, 3) aufweist, die über einen Mittelsteg (4) derart miteinander verbunden sind, dass die Gelenkmanschette (1) im ausgebreiteten Zustand eine leicht V-förmige Gestalt aufweist und dass bei angelegter Manschette das Schienbein sowie der Fussrücken frei bleiben, welche Manschette weiterhin einstückig und aus einem thermoplastischen Kunststoff gebildet ist und deren Laschen zur Befestigung am Bein oberhalb des Sprunggelenks mit einem Befestigungselement verbindbar sind, dadurch gekennzeichnet, dass die Breite des Mittelsteges (4) über seine gesamte Erstreckung etwas geringer ist als die Breite der Laschen (2, 3), wobei der Mittelsteg im angelegten Zustand der Manschette im wesentlichen in der Fusskehle anzuordnen ist, und dass der thermoplastische Kunststoff von einem am Gelenk verformbaren Niedertemperatur-Thermoplast gebildet ist.

2. Gelenkmanschette nach Anspruch 1, dadurch gekennzeichnet, dass die Breite des Mittelsteges (4) 3 bis 5 cm beträgt.

3. Gelenkmanschette nach Anspruch 1 oder 2, dadurch gekennzeichnet, dass die Breite der Laschen (2, 3) 6 bis 11 cm beträgt.

4. Gelenkmanschette nach einem der Ansprüche 1 bis 3, dadurch gekennzeichnet, dass die Laschen an der Medialseite bis 10 cm oberhalb des Innenknöchels und an der Aussenseite bis ca. 11,5 oberhalb des Aussenknöckels reichen.

5. Gelenkmanschette nach einem der Ansprüche 1 bis 4, dadurch gekennzeichnet, dass auf mindestens der Innenfläche der Gelenkmanschette eine Polsterschicht (6) aufgebracht ist.

6. Gelenkmanschette nach Anspruch 5, dadurch gekennzeichnet, dass die Polsterschicht aus Schaumstoff besteht.

7. Gelenkmanschette nach einem der Ansprüche 1 bis 6, dadurch gekennzeichnet, dass die Gelenkmanschette mit einer Hülle (7) aus Leder, Kunststoff oder Stoff bezogen ist.

8. Gelenkmanschette nach einem der Ansprüche 1 bis 7, dadurch gekennzeichnet, dass die Gelenkmanschette integraler Bestandteil eines Schuhes ist.

## Claims

1. A joint sleeve for supporting and guiding the ankle joint during sporting activity, which sleeve has two flaps (2, 3) for covering the ankle joint at the sides, which flaps are connected to one another by way of a middle portion (4) in such a way that, when it is opened out, the joint sleeve has a slightly V-shaped form and, when it is fitted, the shin and the dorsum of the foot remain uncovered, the sleeve being made in one piece from a synthetic thermoplastics material, and its flaps can be connected to a fastening element for fastening it to the leg above the ankle joint, characterised in that the width of the middle portion (4) over its entire length is somewhat smaller than the width of the flaps (2, 3), wherein, when fitted, the middle portion of the sleeve should be disposed substantially in the arch of the foot, and the thermoplastic synthetic material is formed by a low-temperature thermoplastic which can be shaped on the joint.

2. A joint sleeve as claimed in claim 1, characterised in that the width of the middle portion (4) is 3 to 5 cm.

3. A joint sleeve as claimed in claim 1 or 2, characterised in that the width of the flaps (2, 3) is 6 to 11 cm.

4. A joint sleeve as claimed in any of claims 1 to 3, characterised in that the flaps on the medial side extend up to 10 cm above the inside of the ankle and those on the outside extend up to approximately 11.5 cm above the outer ankle.

5. A joint sleeve as claimed in any of claims 1 to 4, characterised in that a padded layer (6) is provided on at least the inside of the joint sleeve.

6. A joint sleeve as claimed in claim 5, characterised in that the padded layer is made of foam.

7. A joint sleeve as claimed in any of claims 1 to 6, characterised in that the joint sleeve is covered by a cover (7) made of leather, synthetic material or fabric.

8. A joint sleeve as claimed in any of claims 1 to 7, characterised in that the joint sleeve is an integral part of a shoe.

## Revendications

1. Gaine d'articulation pour soutenir et guider l'articulation du pied dans l'exercice du sport, qui comporte, pour recouvrir latéralement l'articulation du pied, deux oreilles (2, 3), reliées ensemble par une partie centrale (4) de telle façon que la gaine d'articulation (1) présente, à l'état étalé, une forme légèrement en V et que, la gaine étant mise en place, le tibia et le dessus du pied restent libres, ladite gaine étant en outre d'une seule pièce et en matière synthétique thermoplastique, et ses oreilles pouvant être reliées par un élément d'attache pour la fixation sur la jambe au-dessus de l'articulation du pied, ladite gaine étant caractérisée en ce que la largeur de la partie centrale (4) est un peu moindre, sur toute son étendue, que la largeur des oreilles (2, 3), ladite partie centrale étant disposée sensiblement dans la voûte plantaire, quand la gaine est en place, et en ce que la matière synthétique thermoplastique est formée par une matière thermoplastique qui peut se déformer en s'adaptant à l'articulation à basse température.

2. Gaine d'articulation selon la revendication 1, caractérisée en ce que la largeur de la partie centrale (4) est de 3 à 5 cm.

3. Gaine d'articulation selon la revendication 1 ou 2, caractérisée en ce que la largeur des oreilles (2, 3) est de 6 à 11 cm.

4. Gaines d'articulation selon l'une des revendications 1 à 3, caractérisée en ce que du côté médian les oreilles atteignent jusqu'à 10 cm au-dessus de la malléole intérieure et du côté extérieur, jusqu'à 11,5 cm environ au-dessus de la malléole extérieure.

5. Gaine d'articulation selon l'une des revendications 1 à 4, caractérisée en ce qu'une couche de rembourrage (6) est apposée au moins sur la surface intérieure de la gaine d'articulation.

6. Gaine d'articulation selon la revendication 5, caractérisée en ce que la couche de rembourrage est en matière mousse.

7. Gaine d'articulation selon l'une des revendications 1 à 6, caractérisée en ce que ladite gaine est revêtue d'une enveloppe (7) en cuir, matière synthétique ou tissu.

8. Gaine d'articulation selon l'une des revendications 1 à 7, caractérisée en ce que la gaine d'articulation fait partie constituante intégrale d'une chaussure.

Fig. 1

Fig. 2

Fig. 3